# FASCICULE DE BREVET EUROPEEN

(11) **EP 1 703 888 B1**
(45) Date de publication et mention de la délivrance du brevet: **05.10.2011**
(21) Numéro de dépôt: 04816451.1
(22) Date de dépôt: 21.12.2004
(51) Int. Cl.: A61K 8/04, A61Q 5/02, A61Q 5/12, A61K 8/20, A61K 8/44

(54) **COMPOSITION COSMETIQUE DE TYPE EMULSION EAU-DANS-EAU A BASE DE TENSIOACTIFS ET DE POLYMERES CATIONIQUES**
KOSMETISCHE ZUSAMMENSETZUNG VOM WASSER/WASSER-EMULSIONSTYP AUF BASIS VON TENSIDEN UND KATIONISCHEN POLYMEREN
COSMETIC COMPOSITION OF THE WATER-IN-WATER TYPE EMULSION BASED ON SURFACTANTS AND CATIONIC POLYMERS

(30) Priorité: 05.01.2004 FR 0400026; 02.02.2004 US 540329 P
(43) Date de publication de la demande: 27.09.2006
(73) Titulaire: L'Oréal, 75008 Paris (FR)
(72) Inventeur: SIMONET, Frédéric, F-77131 Touquin (FR); NICOLAS-MORGANTINI, Luc, F-60810 Rully (FR)
(74) Mandataire: Casalonga, Axel
(86) Numéro de dépôt international: PCT/FR2004/003318
(87) Numéro de publication internationale: WO 2005/074869

(56) Documents cités:
- EP-A- 1 025 839
- WO-A-02/22091
- US-A- 4 940 576

## Description

La présente invention concerne une composition cosmétique de type émulsion eau-dans-eau, comprenant une association particulière tensioactif(s)/polymère(s) cationique(s)/sel(s) hydrosoluble(s), l'utilisation de ladite composition pour le lavage et le conditionnements des cheveux, et un procédé de traitement cosmétique des matières kératiniques mettant en oeuvre cette composition.

Dans le domaine de la cosmétique, lorsqu'on mélange un tensioactif anionique et un polymère cationique en solution aqueuse, on observe généralement une séparation de phase associative. En effet, il se forme un complexe électrostatique avec les deux composés cationique et anionique, lequel complexe est insoluble et conduit à la formation d'un précipité. Un tiers composé, tel qu'un tensioactif amphotère, un alcool ou un sel, peut être ajouté pour éviter la formation du complexe et obtenir un système monophasique limpide.

Par ailleurs, lorsqu'on mélange deux polymères en solution aqueuse, on observe une séparation de phase ségrégative, c'est-à-dire qu'il se forme deux phases enrichies chacune en l'un des deux polymères, l'eau se répartissant entre les deux phases. Cette séparation est due à l'incompatibilité thermodynamique entre les deux polymères et elle est d'autant plus importante que les concentrations ou les masses molaires des polymères sont élevées.

Il n'existe donc aucun système permettant d'obtenir une phase dispersée liquide, enrichie en polymère cationique, dans un milieu tensioactif, notamment anionique.

La demanderesse a découvert de manière surprenante, que l'on pouvait obtenir des gouttelettes enrichies en polymère cationique dans un milieu tensioactif en mélangeant certaines quantités de polymère cationique et de sel hydrosoluble dans un milieu tensioactif. L'addition du sel hydrosoluble permet d'éviter la formation de complexes insolubles entre le polymère cationique et le(s) tensioactif(s) du milieu. Ce type de système sera dénommé ci-après émulsion eau-dans-eau.

Les gouttelettes présentes dans cette émulsion ont une taille moyenne supérieure à 0,1 µm et de préférence inférieure à 100 µm telle que mesurée par microscopie optique.

En outre, cette émulsion permet d'obtenir un nouveau mode de vectorisation des polymères cationiques et des agents cosmétiques sur les fibres kératiniques.

L'émulsion selon l'invention présente aussi de meilleures qualités d'écoulement, c'est-à-dire qu'elle ne s'écoule pas par paquets, ce qui est apprécié des consommateurs.

L'invention a donc pour objet une composition cosmétique de type émulsion eau-dans-eau, comprenant dans un milieu aqueux cosmétiquement acceptable, au moins un tensioactif, une quantité adaptée d'au moins un sel hydrosoluble et une quantité adaptée d'au moins un polymère cationique de masse moléculaire en poids supérieure à 10⁵, en un rapport pondéral sel(s) hydrosoluble(s)/polymère(s) cationique(s) particulier.

Un autre objet de l'invention est l'utilisation de la composition pour le lavage et le conditionnement des matière kératiniques telles que les cheveux.

L'invention a encore pour objet un procédé de traitement cosmétique des matières kératiniques mettant en oeuvre la composition selon l'invention.

D'autres objets, caractéristiques, aspects et avantages de l'invention apparaîtront encore plus clairement à la lecture de la description et des divers exemples qui suivent.

Selon l'invention, la composition cosmétique de type émulsion eau-dans-eau, comprend dans un milieu aqueux cosmétiquement acceptable,
- 4 à 50% en poids, par rapport au poids total de la composition cosmétique, d'au moins un tensioactif,
- plus de 3% en poids, par rapport au poids total de la composition, d'au moins un sel hydrosoluble minéral ou organique, comportant, lorsqu'il est organique, de 1 à 7 atomes de carbone dans l'anion,
- au moins 0,5 % en poids, par rapport au poids total de la composition, d'au moins un polymère cationique de masse
moléculaire en poids supérieure à 10⁵,
en un rapport pondéral sel(s) hydrosoluble(s)/polymère(s) cationique(s) supérieur ou égal à 4,5, de préférence allant de 4,5 à 19, mieux encore de 4,5 à 15.

Les tensioactifs utilisables dans la composition selon l'invention peuvent être anioniques, amphotères, non ioniques ou cationiques.

A titre de tensioactifs anioniques utilisables, seuls ou en mélanges, dans le cadre de la présente invention, on peut citer notamment les sels, en particulier les sels de métaux alcalins tels que les sels de sodium, les sels d'ammonium, les sels d'amines, les sels d'aminoalcools ou les sels de métaux alcalino-terreux, par exemple, de magnésium, des composés suivants : les sulfates d'alkyle, les alkyléther-sulfates, les alkylamidoéthersulfates, les alkylarylpolyéthersulfates, les monoglycéride-sulfates ; les alkylsulfonates, les phosphates d'alkyle, les alkylamidesulfonates, les alkylarylsulfonates, les α-oléfine-sulfonates, les paraffine-sulfonates ; les sulfosuccinates d'alkyle, les alkyléthersulfosuccinates, les alkylamidesulfosuccinates ; les sulfoacétates d'alkyle ; les acylsarcosinates ; et les acylglutamates, les groupes alkyle ou acyle de tous ces composés comportant de 6 à 24 atomes de carbone et le groupe aryle désignant de préférence un groupe phényle ou benzyle.

On peut également utiliser les esters d'alkyle en C₆-C₂₄ et d'acides polyglycoside-carboxyliques tels que les glucoside-citrates d'alkyle, les polyglycoside-tartrates d'alkyle, et les polyglycoside-sulfosuccinates d'alkyle ; les sulfosuccinamates d'alkyle, les iséthionates d'acyle et les N-acyltaurates, le groupe alkyle ou acyle de tous ces composés comportant de 12 à 20 atomes de carbone. Parmi les tensioactifs anioniques encore utilisables, on peut également citer les lactylates d'acyle dont le groupe acyle comporte de 8 à 20 atomes de carbone.

En outre, on peut encore citer les acides d'alkyl-D-galactoside uroniques et leurs sels ainsi que les acides alkyl(C₆-C₂₄)éthercarboxyliques polyoxyalkylénés, les acides alkyl(C₆-C₂₄)aryl(C₆-C₂₄)éther-carboxyliques polyoxyalkylénés, les acides alkyl(C₆-C₂₄)amidoéther carboxyliques polyoxyalkylénés et leurs sels, en particulier ceux comportant de 2 à 50 groupements oxyde d'éthylène, et leurs mélanges.

Parmi les tensioactifs anioniques cités ci-dessus, on préfère utiliser selon l'invention les sulfates d'alkyle, les alkyléther-sulfates comme le lauryléthersulfate de sodium, de préférence à 2 ou 3 moles d'oxyde d'éthylène, les alkyléthercarboxylates, les groupes alkyle comportant généralement de 6 à 24 atomes de carbone, et de préférence de 8 à 16 atomes de carbone, sous la forme particulière de sels de sodium, de magnésium ou d'ammonium.

Les agents tensioactifs amphotères, convenant dans la présente invention, peuvent être notamment des dérivés d'amines aliphatiques secondaires ou tertiaires, dans lesquels le groupe aliphatique est une chaîne linéaire ou ramifiée comportant de 8 à 22 atomes de carbone et contenant, au moins un groupe anionique hydrosolubilisant tel que, par exemple, un groupe carboxylate, sulfonate, sulfate, phosphate ou phosphonate ; on peut citer encore les alkyl(C₈-C₂₀)bétaïnes, les sulfobétaïnes, les alkyl(C₈-C₂₀)amidoalkyl(C₆-C₈)-bétaïnes ou les alkyl(C₈-C₂₀)amidoalkyl(C₆-C₈)sulfobétaïnes ; et leurs mélanges.

Parmi les dérivés d'amines, on peut citer les produits commercialisés sous la dénomination MIRANOL^{®}, tels que décrits dans les brevets US-2 528 378 et US-2 781 354 et classés dans le dictionnaire CTFA, 3ème édition, 1982, sous les dénominations Amphocarboxyglycinate et Amphocarboxypropionate de structures respectives (1) et (2) :

Rₐ-CONHCH₂CH₂-N⁺(R_{b})(R_{c})(CH₂COO⁻) (1)

dans laquelle :
Rₐ représente un groupe alkyle dérivé d'un acide Rₐ-COOH présent dans l'huile de coprah hydrolysée, un groupe heptyle, nonyle ou undécyle,
R_{b} représente un groupe bêta-hydroxyéthyle, et
R_{c} représente un groupe carboxyméthyle ;
   et

   R_{a'}-CONHCH₂CH₂-N(B)(C) (2)
dans laquelle :
B représente -CH₂CH₂OX',
C représente -(CH₂)_{z}-Y', avec z = 1 ou 2,
X' représente le groupe -CH₂CH₂-COOH ou un atome d'hydrogène,
Y' représente -COOH ou le groupe -CH₂-CHOH-SO₃H,
R_{a'} représente un groupe alkyle d'un acide R_{a'}-COOH présent dans l'huile de coprah ou dans l'huile de lin hydrolysée, un groupe alkyle, notamment en C₁₇ et sa forme iso, un groupe en C₁₇ insaturé.

Ces composés sont classés dans le dictionnaire CTFA, 5ème édition, 1993, sous les dénominations cocoamphodiacétate de disodium, lauroamphodiacétate de disodium, caprylamphodiacétate de disodium, capryloamphodiacétate de disodium, cocoamphodipropionate de disodium, lauroamphodipropionate de disodium, caprylamphodipropionate de disodium, capryloamphodipropionate de disodium, acide lauroamphodipropionique, acide cocoamphodipropionique.

A titre d'exemple, on peut citer le cocoamphodiacétate commercialisé sous la dénomination commerciale MIRANOL^{®} C2M concentré par la société RHODIA.

Parmi les tensioactifs amphotères, on utilise de préférence les alkyl(C₈-C₂₀)bétaïnes, les alkyl(C₈-C₂₀)amidoalkyl(C₆-C₈)bétaïnes, les alkylamphomonoacétates ou alkylamphodiacétates et leurs mélanges.

Les agents tensioactifs non-ioniques utilisables dans la composition selon l'invention, sont des composés bien connus en soi (voir notamment à cet égard "Handbook of Surfactants" par M.R. PORTER, éditions Blackie & Son (Glasgow and London), 1991, pp 116-178). Ainsi, ils peuvent être notamment choisis parmi les alcools, les alpha-diols, les alkyl(C₁-C₂₀)phénols ou les acides gras polyéthoxylés, polypropoxylés ou polyglycérolés, ayant une chaîne grasse comportant, par exemple, de 8 à 18 atomes de carbone, le nombre de groupements oxyde d'éthylène ou oxyde de propylène pouvant aller notamment de 2 à 50 et le nombre de groupements glycérol pouvant aller notamment de 2 à 30. On peut également citer les copolymères d'oxyde d'éthylène et de propylène, les condensats d'oxyde d'éthylène et de propylène sur des alcools gras ; les amides gras polyéthoxylés ayant de préférence de 2 à 30 moles d'oxyde d'éthylène, les amides gras polyglycérolés comportant en moyenne 1 à 5 groupements glycérol et en particulier 1,5 à 4 ; les amines grasses polyéthoxylées ayant de préférence 2 à 30 moles d'oxyde d'éthylène ; les esters d'acides gras du sorbitane éthoxylés ayant de 2 à 30 moles d'oxyde d'éthylène ; les esters d'acides gras du saccharose, les esters d'acides gras du polyéthylèneglycol, les alkyl(C₆-C₂₄)polyglycosides, les dérivés de N-alkyl(C₆-C₂₄)glucamine, les oxydes d'amines tels que les oxydes d'alkyl(C₁₀-C₁₄)amines ou les oxydes de N-acyl(C₁₀-C₁₄)aminopropylmorpholine ; et leurs mélanges.

Parmi les tensioactifs non-ioniques cités ci-dessus, on utilise de préférence les alkyl(C₆-C₂₄)polyglycosides.

A titre de tensioactif cationique, on peut notamment citer cationiques les sels d'amines grasses primaires, secondaires ou tertiaires, éventuellement polyoxyalkylénées ; les sels d'ammonium quaternaire tels que les chlorures ou les bromures de tétraalkylammonium, d'alkylamidoalkyltrialkylammonium, de trialkylbenzylammonium, de trialkylhydroxyalkylammonium ou d'alkylpyridinium ; les dérivés d'imidazoline ; ou les oxydes d'amines à caractère cationique.

De préférence, les compositions de l'invention contiennent au moins un tensioactif anionique associé éventuellement à un ou plusieurs tensioactifs amphotères ou non ioniques.

Avantageusement, ces compositions contiennent au moins un tensioactif anionique et au moins un tensioactif amphotère.

Les tensioactifs sont présents en une quantité totale allant de 4 à 50% en poids, encore plus préférentiellement de 4 à 20% en poids par rapport au poids total de la composition cosmétique.

Les sels hydrosolubles pouvant être utilisés dans la présente invention sont de préférence choisis parmi les sels hydrosolubles de métaux monovalents ou divalents, par exemple de métaux alcalins ou alcalino-terreux, ou d'ammonium ou d'amines et des acides minéraux ou des acides carboxyliques. Les sels organiques comprennent de 1 à 7 atomes de carbone dans l'anion.

A titre d'exemples de tels sels, on peut notamment citer chlorure de sodium, le chlorure de potassium, le chlorure de calcium, le chlorure de magnésium, le chlorure d'ammonium, le chlorure de monoéthanolamine, le citrate de sodium, le citrate d'ammonium, le sulfate de magnésium et les sels de sodium de l'acide phosphorique. De préférence, on utilise les sels de métaux monovalents et le chlorure de sodium est particulièrement préféré.

Par "hydrosoluble", on entend au sens de la présente invention un composé présentant à 25°C et sous pression atmosphérique, une solubilité dans l'eau supérieure ou égale à 1% et de préférence supérieure ou égale à 2,5%.

Les sels hydrosolubles, y compris ceux présents comme adjuvants des matières premières utilisées, sont présents en une quantité strictement supérieure à 3 % en poids, plus préférentiellement allant de 3,1 à 30% en poids, et encore plus préférentiellement de 3,1 à 10% en poids par rapport au poids total de la composition.

Par "polymère cationique", on entend tout polymère contenant des groupements cationiques et/ou des groupements ionisables en groupements cationiques.

Les polymères cationiques utilisables conformément à la présente invention peuvent être choisis parmi tous ceux déjà connus en soi comme améliorant les propriétés cosmétiques des cheveux traités par des compositions détergentes, à savoir notamment ceux décrits dans la demande de brevet EP-A-0 337 354 et dans les demandes de brevets français FR-A- 2 270 846, 2 383 660, 2 598 611, 2 470 596 et 2 519 863.

Les polymères cationiques préférés sont choisis parmi ceux qui contiennent des motifs comportant des groupements amine primaires, secondaires, tertiaires et/ou quaternaires pouvant soit faire partie de la chaîne principale polymère, soit être portés par un substituant latéral directement relié à celle-ci.

Les polymères cationiques utilisés ont une masse moléculaire moyenne en poids supérieure à 10⁵, et de préférence comprise entre 10⁵ et 10⁸.

Parmi les polymères cationiques, on peut citer plus particulièrement les polymères du type polyamine, polyaminoamide et polyammonium quaternaire. Ce sont des produits connus.

Les polymères du type polyamine, polyaminoamide, polyammonium quaternaire, que l'on peut utiliser dans la composition de la présente invention, sont ceux décrits dans les brevets français n^{os} 2 505 348 et 2 542 997. Parmi ces polymères, on peut citer :
(1) les homopolymères ou copolymères dérivés d'esters ou d'amides acryliques ou méthacryliques et comportant au moins un des motifs de formules suivantes: 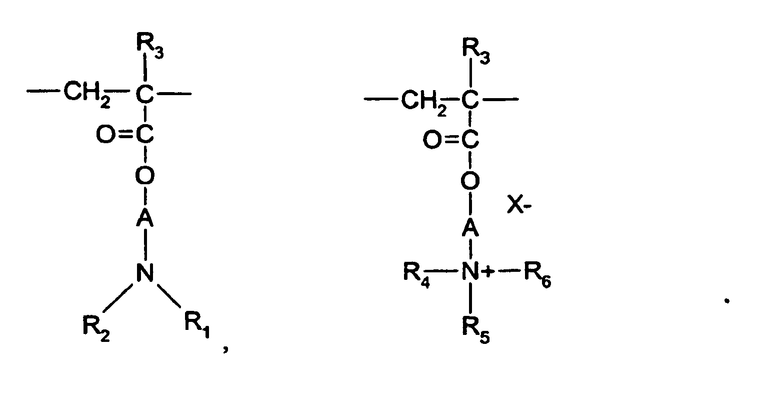 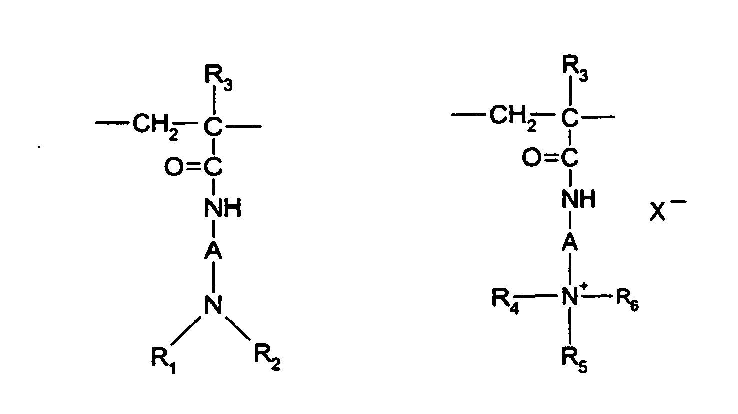 dans lesquelles:
   R₁ et R₂, identiques ou différents, représentent un atome d'hydrogène ou un groupe alkyle ayant de 1 à 6 atomes de carbone, et de préférence un groupe méthyle ou éthyle ;
   R₃, identiques ou différents, désignent un atome d'hydrogène ou un groupe CH₃ ;
   les symboles A, identiques ou différents, représentent un groupe alkyle, linéaire ou ramifié, comportant de 1 à 6 atomes de carbone, de préférence 2 ou 3 atomes de carbone, ou un groupe hydroxyalkyle comportant de 1 à 4 atomes de carbone ;
   R₄, R₅, R₆, identiques ou différents, représentent un groupe alkyle ayant de 1 à 18 atomes de carbone ou un groupe benzyle, et de préférence un groupe alkyle ayant de 1 à 6 atomes de carbone ;
   X désigne un anion dérivé d'un acide minéral ou organique tel qu'un anion méthosulfate ou un halogénure comme le chlorure ou le bromure.
      Les copolymères de la famille (1) peuvent contenir en outre un ou plusieurs motifs dérivant de comonomères pouvant être choisis dans la famille des acrylamides, méthacrylamides, diacétone-acrylamides, acrylamides et méthacrylamides substitués sur l'atome d'azote par des groupes alkyle inférieur (C₁-C₄), des groupes dérivés des acides acryliques ou méthacryliques ou de leurs esters, de vinyllactames tels que la vinylpyrrolidone ou le vinylcaprolactame, d'esters vinyliques.
      Ainsi, parmi ces copolymères de la famille (1), on peut citer :
      - les copolymères d'acrylamide et de méthacrylate de diméthylaminoéthyle quaternisé au sulfate de diméthyle ou avec un halogénure de diméthyle,
      - les copolymères d'acrylamide et de chlorure de méthacryloyloxyéthyltriméthylammonium décrits, par exemple, dans la demande de brevet EP-A-080976,
      - les copolymères d'acrylamide et de méthosulfate de méthacryloyloxyéthyltriméthylammonium,
      - les copolymères vinylpyrrolidone/acrylate ou méthacrylate de dialkylaminoalkyle quaternisés ou non. Ces polymères sont décrits en détail dans les brevets français 2 077 143 et 2 393 573,
      - les terpolymères méthacrylate de diméthylaminoéthyle/ vinylcaprolactame/vinylpyrrolidone,
      - les copolymères vinylpyrrolidone/méthacrylamidopropyl-diméthylamine, et
      - les copolymères vinylpyrrolidone/méthacrylamide de diméthylaminopropyle quaternisé.
   (2) Les dérivés d'éthers de cellulose comportant des groupements ammonium quaternaires décrits dans le brevet français 1 492 597, et en particulier les polymères commercialisés sous les dénominations "JR" (JR 400, JR 125, JR 30M) ou "LR" (LR 400, LR 30M) par la Société Union Carbide Corporation. Ces polymères sont également définis dans le dictionnaire CTFA comme des ammoniums quaternaires d'hydroxyéthylcellulose ayant réagi avec un époxyde substitué par un groupement triméthylammonium.
   (3) Les dérivés de cellulose cationiques tels que les copolymères de cellulose ou les dérivés de cellulose greffés avec un monomère hydrosoluble d'ammonium quaternaire, et décrits notamment dans le brevet US 4 131 576, tels que les hydroxyalkylcelluloses, comme les hydroxyméthyl-, hydroxyéthyl- ou hydroxypropyl-celluloses greffées notamment avec un sel de méthacryloyléthyl-triméthylammonium, de méthacrylamidopropyl-triméthylammonium, de diméthyldiallylammonium.

Les produits commercialisés répondant à cette définition sont plus particulièrement les produits vendus sous la dénomination "Celquat^{®} L 200" et "Celquat^{®} H 100" par la Société National Starch. (4) Les polysaccharides cationiques décrits dans les brevets US 3 589 578 et 4 031 307 tels que les gommes de guar contenant des groupements cationiques trialkylammonium. On utilise, par exemple, des gommes de guar modifiées par un sel, par exemple le chlorure, de 2,3-époxypropyltriméthylammonium.

De tels produits sont commercialisés notamment sous les dénominations commerciales de JAGUAR^{®} C13 S, JAGUAR^{®} C15, JAGUAR^{®} C17 ou JAGUAR^{®} C162 par la société MEYHALL.
(5) Les polymères constitués de motifs pipérazinyle et de groupes divalents alkylène ou hydroxyalkylène à chaînes droites ou ramifiées, éventuellement interrompues par des atomes d'oxygène, de soufre, d'azote ou par des cycles aromatiques ou hétérocycliques, ainsi que les produits d'oxydation et/ou de quaternisation de ces polymères. De tels polymères sont notamment décrits dans les brevets français 2 162 025 et 2 280 361.
(6) Les polyaminoamides solubles dans l'eau, préparés en particulier par polycondensation d'un composé acide avec une polyamine ; ces polyaminoamides peuvent être réticulés par une épihalohydrine, un diépoxyde, un dianhydride, un dianhydride non saturé, un dérivé bis-insaturé, une bis-halohydrine, un bis-azétidinium, une bis-haloacyldiamine, un bis-halogénure d'alkyle ou encore par un oligomère résultant de la réaction d'un composé bifonctionnel réactif vis-à-vis d'une bis-halohydrine, d'un bis-azétidinium, d'une bis-haloacyldiamine, d'un bis-halogénure d'alkyle, d'une épilhalohydrine, d'un diépoxyde ou d'un dérivé bis-insaturé ; l'agent réticulant étant utilisé dans des proportions allant de 0,025 à 0,35 mole par groupement amine du polyaminoamide ; ces polyaminoamides peuvent être alkylés ou s'ils comportent une ou plusieurs fonctions amines tertiaires, quaternisées. De tels polymères sont notamment décrits dans les brevets français 2 252 840 et 2 368 508.
(7) Les dérivés de polyaminoamides résultant de la condensation de polyalkylènes-polyamines avec des acides polycarboxyliques, suivie d'une alkylation par des agents bifonctionnels. On peut citer, par exemple, les polymères acide adipique/diakylaminohydroxyalkyl-dialkylènetriamine dans lesquels le groupe alkyle comporte de 1 à 4 atomes de carbone et désigne de préférence un groupe méthyle, éthyle, propyle, et le groupe alkylène comporte de 1 à 4 atomes de carbone, et désigne de préférence le groupe éthylène. De tels polymères sont notamment décrits dans le brevet français 1 583 363.

Parmi ces dérivés, on peut citer plus particulièrement les polymères acide adipique/diméthylaminohydroxypropyl-diéthylène-triamine.
(8) Les polymères obtenus par réaction d'une polyalkylène-polyamine comportant deux groupements amine primaire et au moins un groupement amine secondaire, avec un acide dicarboxylique choisi parmi l'acide diglycolique et les acides dicarboxyliques aliphatiques saturés ayant de 3 à 8 atomes de carbone. Le rapport molaire entre la polyalkylène-polylamine et l'acide dicarboxylique étant compris entre 0,8 : 1 et 1,4 : 1 ; le polyaminoamide en résultant étant amené à réagir avec l'épichlorhydrine dans un rapport molaire d'épichlorhydrine par rapport au groupement amine secondaire du polyaminoamide compris entre 0,5 : 1 et 1,8 : 1. De tels polymères sont notamment décrits dans les brevets américains 3 227 615 et 2 961 347.
(9) Les cyclopolymères d'alkyldiallylamine ou de dialkyldiallylammonium tels que les homopolymères ou copolymères comportant, comme constituant principal de la chaîne, des motifs répondant aux formules (Va) ou (Vb) : 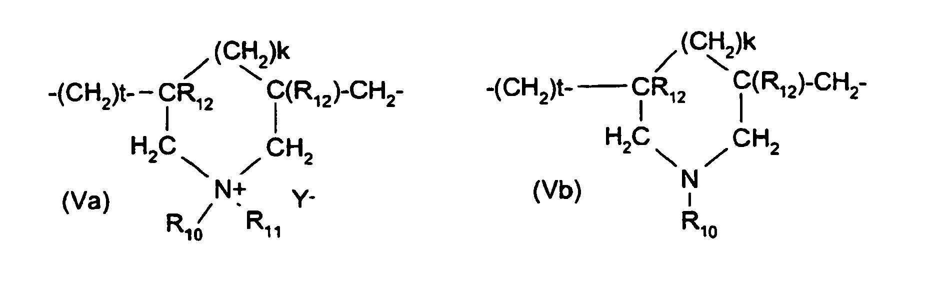
dans lesquelles k et t sont égaux à 0 ou 1, la somme k + t étant égale à 1 ; R₁₂ désigne un atome d'hydrogène ou un groupe méthyle ; R₁₀ et R₁₁, indépendamment l'un de l'autre, désignent un groupement alkyle ayant de 1 à 6 atomes de carbone, un groupement hydroxyalkyle dans lequel le groupement alkyle a de préférence 1 à 5 atomes de carbone, un groupement amidoalkyle inférieur (C₁-C₄), ou alors R₁₀ et R₁₁ peuvent désigner conjointement avec l'atome d'azote auquel ils sont rattachés, des groupements hétérocycliques, tels que pipéridinyle ou morpholinyle ; Y⁻ est un anion tel que bromure, chlorure, acétate, borate, citrate, tartrate, bisulfate, bisulfite, sulfate, phosphate. Ces polymères sont notamment décrits dans le brevet français 2 080 759 et dans son certificat d'addition 2 190 406.

R₁₀ et R₁₁, indépendamment l'un de l'autre, désignent de préférence un groupement alkyle ayant de 1 à 4 atomes de carbone.

Parmi les polymères définis ci-dessus, on peut citer plus particulièrement l'homopolymère de chlorure de diméthyldiallylammonium vendu sous la dénomination "MERQUAT^{®} 100" par la société CALGON (et ses homologues de faibles masses moléculaires moyenne en poids) et les copolymères de chlorure de diallyldiméthylammonium et d'acrylamide commercialisés sous la dénomination "MERQUAT^{®} 550".
(10) Les polymères de diammonium quaternaire contenant des motifs récurrents répondant à la formule (VI) : 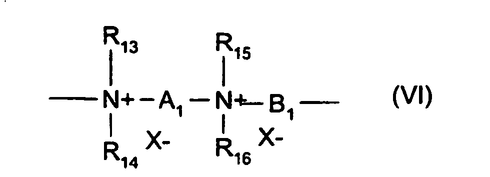 dans laquelle :
   R₁₃, R₁₄, R₁₅ et R₁₆, identiques ou différents, représentent des groupes aliphatiques, alicycliques ou arylaliphatiques contenant de 1 à 20 atomes de carbone ou des groupes hydroxyalkylaliphatiques inférieurs, ou bien R₁₃, R₁₄, R₁₅ et R₁₆, ensemble ou séparément, constituent avec les atomes d'azote auxquels ils sont rattachés des hétérocycles contenant éventuellement un second hétéroatome autre que l'azote, ou bien R₁₃, R₁₄, R₁₅ et R₁₆ représentent un groupe alkyle en C₁-C₆, linéaire ou ramifié, substitué par un groupement nitrile, ester, acyle, amide ou -CO-O-R₁₇-D ou -CO-NH-R₁₇-D où R₁₇ est un groupe alkylène et D un groupement ammonium quaternaire ;
   A₁ et B₁ représentent des groupements polyméthyléniques contenant de 2 à 20 atomes de carbone, pouvant être linéaires ou ramifiés, saturés ou insaturés, et pouvant contenir, liés à ou intercalés dans la chaîne principale, un ou plusieurs cycles aromatiques, ou un ou plusieurs atomes d'oxygène, de soufre ou des groupements sulfoxyde, sulfone, disulfure, amino, alkylamino, hydroxyde, ammonium quaternaire, uréido, amide ou ester, et
   X⁻ désigne un anion dérivé d'un acide minéral ou organique;
   A₁, R₁₃ et R₁₅ peuvent former avec les deux atomes d'azote auxquels ils sont rattachés un cycle pipérazinique ; en outre, si A₁ désigne un groupe alkylène ou hydroxyalkylène linéaire ou ramifié, saturé ou insaturé, B₁ peut également désigner un groupement :

      -(CH₂)ₙ-CO-D-OC-(CH₂)ₙ-
   dans lequel D désigne :
   a) un reste de glycol de formule -O-Z-O-, où Z désigne un groupe hydrocarboné linéaire ou ramifié, ou un groupement répondant à l'une des formules suivantes :

      -(CH₂-CH₂-O)ₓ-CH₂-CH₂-

      -[CH₂-CH(CH₃)-O]_{y}-CH₂-CH(CH₃)-où x et y désignent un nombre entier de 1 à 4, représentant un degré de polymérisation défini et unique ou un nombre quelconque de 1 à 4 représentant un degré de polymérisation moyen ;
   b) un reste de diamine bis-secondaire tel qu'un dérivé de pipérazine ;
   c) un reste de diamine bis-primaire de formule -NH-Y-NH-, où Y désigne un groupe hydrocarboné linéaire ou ramifié, ou bien le groupe divalent -CH₂-CH₂-S-S-CH₂-CH₂- ;
   d) un groupement uréylène de formule -NH-CO-NH- .

De préférence, X⁻ est un anion tel que le chlorure ou le bromure.

Des polymères de ce type sont notamment décrits dans les brevets français 2 320 330, 2 270 846, 2 316 271, 2 336 434 et 2 413 907 et les brevets US 2 273 780, 2 375 853, 2 388 614, 2 454 547, 3 206 462, 2 261 002, 2 271 378, 3 874 870, 4 001 432, 3 929 990, 3 966 904, 4 005 193, 4 025 617, 4 025 627, 4 025 653, 4 026 945 et 4 027 020.

On peut utiliser plus particulièrement les polymères qui sont constitués de motifs récurrents répondant à la formule : 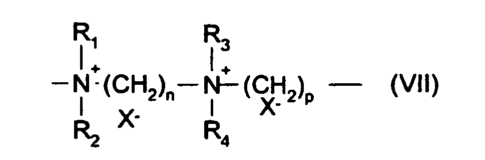 dans laquelle R₁, R₂, R₃ et R₄, identiques ou différents, désignent un groupe alkyle ou hydroxyalkyle ayant de 1 à 4 atomes de carbone environ, n et p sont des nombres entiers variant de 2 à 20 environ et, X- est un anion dérivé d'un acide minéral ou organique.
(11) Les polymères de polyammonium quaternaire constitués de motifs de formule (VIII) : 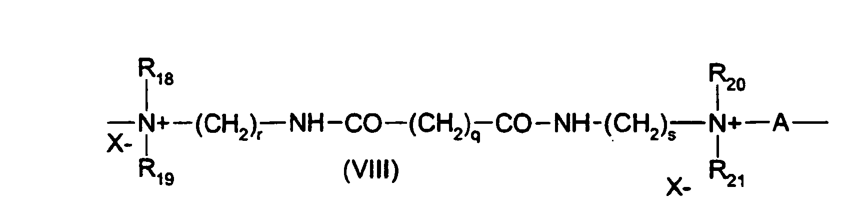 dans laquelle :
   R₁₈, R₁₉, R₂₀ et R₂₁, identiques ou différents, représentent un atome d'hydrogène ou un groupe méthyle, éthyle, propyle, β-hydroxyéthyle, β-hydroxypropyle ou -CH₂CH₂(OCH₂CH₂)ₚOH, où p est égal à 0 ou à un nombre entier compris entre 1 et 6, sous réserve que R₁₈, R₁₉, R₂₀ et R₂₁ ne représentent pas simultanément un atome d'hydrogène,
   r et s, identiques ou différents, sont des nombres entiers compris entre 1 et 6,
   q est égal à 0 ou à un nombre entier compris entre 1 et 34,
   X⁻ désigne un anion tel qu'un halogénure,
   A désigne un radical d'un dihalogénure ou représente de préférence -CH₂-CH₂-O-CH₂-CH₂-.

De tels composés sont notamment décrits dans la demande de brevet EP-A-122 324.
(12) Les polymères quaternaires de vinylpyrrolidone et de vinylimidazole.
(13) Les polymères réticulés de sels de méthacryloyloxyalkyl(C₁-C₄) trialkyl(C₁-C₄)ammonium tels que les polymères obtenus par homopolymérisation du méthacrylate de diméthylaminoéthyle quaternisé par le chlorure de méthyle, ou par copolymérisation de l'acrylamide avec le méthacrylate de diméthylaminoéthyle quaternisé par le chlorure de méthyle, l'homopolymérisation ou la copolymérisation étant suivie d'une réticulation par un composé à insaturation oléfinique, en particulier le méthylène-bisacrylamide.

D'autres polymères cationiques utilisables dans le cadre de l'invention sont des protéines cationiques ou des hydrolysats de protéines cationiques, des polyalkylèneimines, en particulier des polyéthylèneimines, des polymères contenant des motifs vinylpyridine ou vinylpyridinium, des condensats de polyamines et d'épichlorhydrine, des polyuréylènes quaternaires et les dérivés de la chitine.

Parmi tous les polymères cationiques susceptibles d'être utilisés dans le cadre de la présente invention, on préfère mettre en oeuvre les dérivés d'éther de cellulose comportant des groupements ammonium quaternaires tels que les produits vendus sous la dénomination "JR 400" par la Société UNION CARBIDE CORPORATION, les cyclopolymères cationiques, en particulier les homopolymères ou copolymères de chlorure de diméthyldiallylammonium, vendus sous les dénominations MERQUAT^{®} 100, MERQUAT^{®} 550 et MERQUAT^{®} S par la société CALGON, les gommes de guar modifiées par un sel de 2,3-époxypropyl-triméthylammonium, les polymères quaternaires de vinylpyrrolidone et de vinylimidazole.

Les polymères cationiques sont présents de préférence en une quantité allant de 0,5 à 10% en poids, mieux encore de 0,5 à 4% en poids par rapport au poids total de la composition.

Par milieu cosmétiquement acceptable, on entend un milieu compatible avec les matières kératiniques, telles les cheveux et la peau, mais aussi d'odeur, d'aspect et de toucher agréables.

Le milieu aqueux cosmétiquement acceptable est constitué d'eau ou d'un mélange d'eau et d'au moins un solvant cosmétiquement acceptable choisi parmi les alcools inférieurs en C₁-C₄, tels que l'éthanol, l'isopropanol, le tertio-butanol ou le n-butanol ; les polyols tels que le glycérol, le propylèneglycol et les polyéthylèneglycols ; et leurs mélanges.

Le pH des compositions selon l'invention est généralement compris entre 2 et 11, et de préférence entre 3 et 10.

La composition selon l'invention peut comprendre en outre un ou plusieurs additifs classiques bien connus dans la technique, tels que des silicones volatiles ou non, linéaires, ramifiées ou cycliques organomodifiées ou non ; des épaississants ou régulateurs de viscosité, naturels ou synthétiques ; des alcools gras en C₁₂-C₃₀ ; des cires telles que les cires végétales ou des céramides ; des esters gras huileux tels que le myristate d'isopropyle ou les triglycérides; des huiles minérales ou synthétiques telles que les α-oléfines ; des vitamines ou provitamines ; des agents nacrants ; des agents de stabilisation du pH, des conservateurs ; et des colorants.

L'homme de métier veillera à choisir les éventuels additifs et leur quantité de manière à ce qu'ils ne nuisent pas aux propriétés des compositions de la présente invention.

Ces additifs sont généralement présents dans la composition selon l'invention en une quantité allant de 0 à 20 % en poids par rapport au poids total de la composition.

Les compositions selon l'invention peuvent être préparées à température ambiante, à savoir à une température de l'ordre de 20 à 25 °C. On verse la solution de polymère cationique dans la solution de tensioactif(s).

Les compositions conformes à l'invention peuvent être utilisées pour le lavage et/ou le conditionnement des matières kératiniques, en particulier des cheveux, par exemple comme shampoings conditionneurs.

Un autre objet de l'invention est un procédé de traitement cosmétique des matières kératiniques, telles que les cheveux, qui consiste à appliquer une quantité efficace d'une composition telle que décrite ci-dessus, sur lesdites matières, à rincer après un éventuel temps de pose.

Les exemples suivants illustrent la présente invention. Les quantités indiquées ci-après sont exprimées en pourcentage en poids par rapport au poids total de la composition.

### EXEMPLES

Les compositions 1 à 3, selon l'invention, ont été préparées à partir des ingrédients indiqués dans le tableau ci-dessous.

Les pourcentages indiqués dans le tableau ci-dessous sont exprimés en pourcentage de matières actives.

| Composition | 1 | 2 | 3 |
|---|---|---|---|
| Lauryléthersulfate de sodium (2,2 moles d'oxyde d'éthylène) | 5 % | 12,5 % | 4,3 % |
| Laurylbétaïne | - | 2,5 % | - |
| Cocoylamidopropylbétaïne | 10% | - | 8,6 % |
| Poly(chlorure de diméthyldiallyl-ammonium)⁽¹⁾ Mp = 4.10⁵ | 1 % | - | 0,4 % |
| Poly(chlorure de diméthyldiallyl-ammonium/acrylamide) ⁽²⁾ Mp = 5.10⁶ | - | 0,5 % | - |
| Guar modifié cationique⁽³⁾ Mp = 1,2.10⁶ | - | - | 0,3 % |
| NaCl | 5,7 % | 6,7 % | 3,3 % |
| Eau qsp | 100 % | 100 % | 100 % |
| pH (ajusté avec HCl concentré) | 7 | 7 | 5,1 |

| | | | |
|---|---|---|---|
| ⁽¹⁾ vendu sous la dénomination commerciale Merquat^{®} 100 par la société NALCO. ⁽²⁾ vendu sous la dénomination commerciale Merquat^{®} 550 par la société NALCO. ⁽³⁾ vendu sous la dénomination commerciale Jaguar^{®} C13S par RHODIA CHIMIE. | | | |

Après mélange des ingrédients, il se forme des dispersions de gouttelettes contenant le(s) polymère(s) cationique(s), présentant une taille de l'ordre de 10 µm, au sein de la matrice tensioactive.

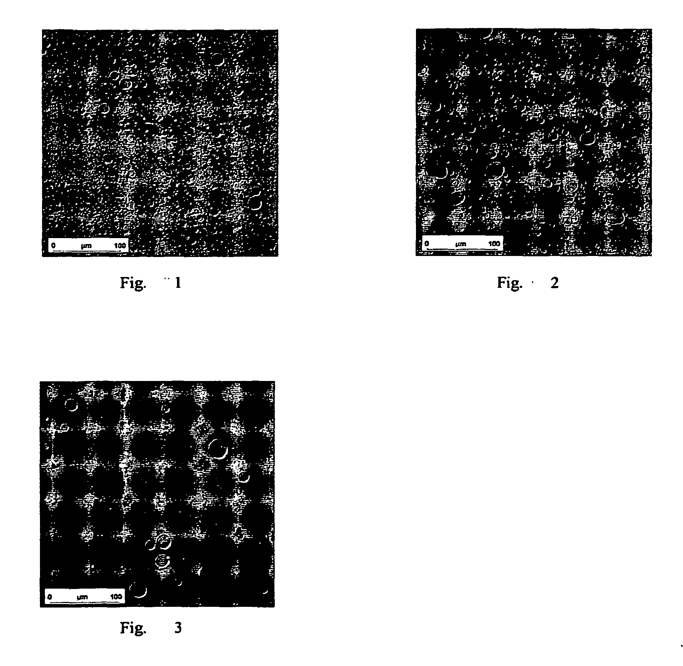

Des photos faites au microscope optique Zeiss, Axioplan 2, grossissement de 20) ont été prises pour les trois dispersions (voir Fig. 1 à 3).

La Figure 1 correspond à la photo de la dispersion de l'exemple No 1,
la Figure 2 correspond à la photo de la dispersion de l'exemple No 2, et
la Figure 3 correspond à la photo de la dispersion de l'exemple No 3.

## Revendications

1. Composition cosmétique de type émulsion eau-dans-eau, comprenant dans un milieu aqueux cosmétiquement acceptable, 4 à 50% en poids, par rapport au poids total de la composition cosmétique, d'au moins un tensioactif,
plus de 3% en poids, par rapport au poids total de la composition, d'au moins un sel hydrosoluble minéral ou organique comportant, lorsqu'il est organique, de 1 à 7 atomes de carbone dans l'anion,
au moins 0,5 % en poids, par rapport au poids total de la composition, d'au moins un polymère cationique de masse moléculaire en poids supérieure à 10⁵,
en un rapport pondéral sel(s) hydrosoluble(s)/polymère(s) cationique(s) supérieur ou égal à 4,5.

2. Composition cosmétique selon la revendication 1, **caractérisée en ce que** le rapport pondéral sel(s) hydrosoluble(s)/polymère(s) cationique(s) est compris entre 4,5 et 19, de préférence entre 4,5 et 15.

3. Composition cosmétique selon la revendication 1 ou 2, **caractérisée en ce que** le tensioactif est anionique, amphotère, nos ionique ou cationique.

4. Composition cosmétique selon la revendication 3, **caractérisée en ce qu'**elle comprend au moins un tensioactif anionique associé éventuellement à un ou plusieurs tensioactifs amphotères ou non ioniques.

5. Composition cosmétique selon la revendication 4, **caractérisée en ce qu'**elle comprend au moins un tensioactif anionique et au moins un tensioactif amphotère.

6. Composition cosmétique selon l'une quelconque des revendications 3 à 5, **caractérisée en ce que** le tensioactif anionique est choisi parmi les sels de métaux alcalins, les sels d'ammonium, les sels d'amines, les sels d'aminoalcools ou les sels de métaux alcalino-terreux, des composés suivants : les sulfates d'alkyle, les alkyléthersulfates, les alkylamidoéthersulfates, les alkylarylpolyéthersulfates, les monoglycéride-sulfates ; les alkylsulfonates, les phosphates d'alkyle, les alkylamidesulfonates, les alkylarylsulfonates, les α-oléfine-sulfonates, les paraffine-sulfonates ; les sulfosuccinates d'alkyle, les alkyléthersulfosuccinates, les alkylamidesulfosuccinates ; les sulfoacétates d'alkyle ; les acylsarcosinates ; et les acylglutamates, les groupes alkyle ou acyle de tous ces composés comportant de 6 à 24 atomes de carbone et le groupe aryle désignant de préférence un groupe phényle ou benzyle ; les esters d'alkyle en C₆-C₂₄ et d'acides polyglycoside-carboxyliques ; les sulfosuccinamates d'alkyle, les iséthionates d'acyle et les N-acyltaurates, le groupe alkyle ou acyle de tous ces composés comportant de 12 à 20 atomes de carbone.

7. Composition cosmétique selon la revendication 6, **caractérisée en ce que** le tensioactif anionique est choisi parmi les sulfates d'alkyle, les alkyléther-sulfates, de préférence à 2 ou 3 moles d'oxyde d'éthylène, et les alkyléthercarboxylates, les groupes alkyle comportant de 6 à 24 atomes de carbone, sous la forme de sels de sodium, de magnésium ou d'ammonnium.

8. Composition cosmétique selon l'une quelconque des revendications 3 à 5, **caractérisée en ce que** le tensioactif amphotère est choisi parmi les alkyl(C₈-C₂₀)bétaïnes, les alkyl(C₈-C₂₀)amidoalkyl(C₆-C₈)bétaïnes, les alkylamphomonoacétates et les alkylamphodiacétates.

9. Composition cosmétique selon l'une quelconque des revendications précédentes, **caractérisée en ce qu'**elle comprend le(s) tensioactif(s) en une quantité allant de 4 à 20 % en poids, par rapport au poids total de la composition.

10. Composition cosmétique selon l'une quelconque des revendications précédentes, **caractérisée en ce que** le sel hydrosoluble est choisi parmi les sels hydrosolubles de métaux monovalents ou divalents, ou d'ammonium ou d'amine, et des acides minéraux ou des acides organiques carboxyliques.

11. Composition cosmétique selon la revendication 10, **caractérisée en ce que** le sel hydrosoluble est choisi parmi le chlorure de sodium, le chlorure de potassium, le chlorure de calcium, le chlorure de magnésium, le chlorure d'ammonium, le chlorure de monoéthanolamine, le citrate de sodium, le citrate d'ammonium, le sulfate de magnésium et les sels de sodium de l'acide phosphorique.

12. Composition cosmétique selon l'une quelconque des revendications précédentes, **caractérisée en ce qu'**elle comprend le(s) sel(s) hydrosoluble(s) en une quantité allant de 3,1 à 30% en poids par rapport au poids total de la composition.

13. Composition cosmétique selon l'une quelconque des revendications précédentes, **caractérisée en ce que** la masse moléculaire moyenne en poids du polymère cationique est comprise entre 10⁵ et 10⁸.

14. Composition selon l'une quelconque des revendications précédentes, **caractérisée en ce que** le polymère cationique est choisi parmi les dérivés d'éther de cellulose comportant des groupements ammonium quaternaires, les cyclopolymères cationiques, les gommes de guar modifiées par un sel de 2,3-époxypropyl-triméthylammonium et les polymères quaternaires de vinylpyrrolidone et de vinylimidazole.

15. Composition selon l'une quelconque des revendications précédentes, **caractérisée en ce qu'**elle comprend le(s) polymère(s) cationique(s) en une quantité allant de 0,5 à 10% en poids par rapport au poids total de la composition.

16. Composition selon l'une quelconque des revendications précédentes, **caractérisée en ce que** le milieu aqueux cosmétiquement acceptable est constitué d'eau ou d'un mélange d'eau et d'au moins un solvant organique.

17. Composition selon l'une quelconque des revendications précédentes, **caractérisée en ce qu'**elle comprend en outre au moins un additif choisi parmi des silicones volatiles ou non, linéaires, ramifiées ou cycliques organomodifiées ou non ; des épaississants ou régulateurs de viscosité, naturels ou synthétiques ; des alcools gras en C₁₂-C₃₀ ; des cires ; des esters gras huileux ; des huiles minérales ou synthétiques ; des vitamines ou provitamines ; des agents nacrants ; des agents de stabilisation du pH, des conservateurs ; et des colorants.

18. Utilisation de la composition selon l'une quelconque des revendications, pour le lavage et/ou le conditionnement des matières kératiniques.

19. Procédé de traitement cosmétique des matières kératiniques, comprenant l'application d'une quantité efficace d'une composition selon l'une quelconque des revendications 1 à 17, sur lesdites matières, à rincer après un éventuel temps de pose.

## Claims

1. Cosmetic composition of water-in-water emulsion type comprising, in a cosmetically acceptable aqueous medium:
4% to 50% by weight, relative to the total weight of the composition, of at least one surfactant, more than 3% by weight, relative to the total weight of the composition, of at least one water-soluble mineral or organic salt comprising, when it is organic, from 1 to 7 carbon atoms in the anion,
at least 0.5% by weight, relative to the total weight of the composition, of at least one cationic polymer with a weight-average molecular mass of greater than 10⁵,
in a water-soluble salt(s)/cationic polymer(s) weight ratio of greater than or equal to 4.5.

2. Cosmetic composition according to Claim 1, **characterized in that** the water-soluble salt(s)/cationic polymer(s) weight ratio is between 4.5 and 19 and preferably between 4.5 and 15.

3. Cosmetic composition according to Claim 1 or 2, **characterized in that** the surfactant is anionic, amphoteric, nonionic or cationic.

4. Cosmetic composition according to Claim 3, **characterized in that** it comprises at least one anionic surfactant optionally combined with one or more amphoteric or nonionic surfactants.

5. Cosmetic composition according to Claim 4, **characterized in that** it comprises at least one anionic surfactant and at least one amphoteric surfactant.

6. Cosmetic composition according to any one of Claims 3 to 5, **characterized in that** the anionic surfactant is chosen from the alkali metal salts, ammonium salts, amine salts, amino alcohol salts or alkaline-earth metal salts of the following compounds: alkyl sulfates, alkyl ether sulfates, alkylamido ether sulfates, alkylarylpolyether sulfates, monoglyceride sulfates; alkyl sulfonates, alkyl phosphates, alkylamide sulfonates, alkylaryl sulfonates, α-olefin sulfonates, paraffin sulfonates; alkyl sulfosuccinates, alkyl ether sulfosuccinates, alkylamide sulfosuccinates; alkyl sulfoacetates; acyl sarcosinates; and acyl glutamates, the alkyl or acyl groups of all these compounds containing from 6 to 24 carbon atoms and the aryl group preferably denoting a phenyl or benzyl group; C₆-C₂₄ alkyl esters of polyglycosidecarboxylic acids; alkyl sulfosuccinamates, acyl isethionates and N-acyltaurates, the alkyl or acyl group of all these compounds containing from 12 to 20 carbon atoms.

7. Cosmetic composition according to Claim 6, **characterized in that** the anionic surfactant is chosen from alkyl sulfates, alkyl ether sulfates, preferably containing 2 or 3 mol of ethylene oxide, and alkyl ether carboxylates, the alkyl groups containing from 6 to 24 carbon atoms, in the form of sodium, magnesium or ammonium salts.

8. Cosmetic composition according to any one of Claims 3 to 5, **characterized in that** the amphoteric surfactant is chosen from (C₈-C₂₀)alkylbetaines, (C₈-C₂₀)alkylamido(C₆-C₈)alkylbetaines, alkylamphomono-acetates and alkylamphodiacetates.

9. Cosmetic composition according to any one of the preceding claims, **characterized in that** it comprises the surfactant(s) in an amount ranging from 4% to 20% by weight relative to the total weight of the composition.

10. Cosmetic composition according to any one of the preceding claims, **characterized in that** the water-soluble salt is chosen from water-soluble salts of monovalent or divalent metals, or of ammonium or of amine, and of mineral acids or of organic carboxylic acids.

11. Cosmetic composition according to Claim 10, **characterized in that** the water-soluble salt is chosen from sodium chloride, potassium chloride, calcium chloride, magnesium chloride, ammonium chloride, monoethanolamine chloride, sodium citrate, ammonium citrate, magnesium sulfate and the sodium salts of phosphoric acid.

12. Cosmetic composition according to any one of the preceding claims, **characterized in that** it comprises the water-soluble salt(s) in an amount ranging from 3.1% to 30% by weight relative to the total weight of the composition.

13. Cosmetic composition according to any one of the preceding claims, **characterized in that** the weight-average molecular mass of the cationic polymer is between 10⁵ and 10⁸.

14. Composition according to any one of the preceding claims, **characterized in that** the cationic polymer is chosen from cellulose ether derivatives comprising quaternary ammonium groups, cationic cyclopolymers, guar gums modified with a 2,3-epoxypropyltrimethylammonium salt and quaternary polymers of vinylpyrrolidone and of vinylimidazole.

15. Composition according to any one of the preceding claims, **characterized in that** it comprises the cationic polymer(s) in an amount ranging from 0.5% to 10% by weight relative to the total weight of the composition.

16. Composition according to any one of the preceding claims, **characterized in that** the cosmetically acceptable aqueous medium consists of water or of a mixture of water and of at least one organic solvent.

17. Composition according to any one of the preceding claims, **characterized in that** it also comprises at least one additive chosen from linear, branched or cyclic, organomodified or non-organomodified, volatile or non-volatile silicones; natural or synthetic thickeners or viscosity regulators; C₁₂-C₃₀ fatty alcohols; waxes; oily fatty esters; mineral or synthetic oils; vitamins or provitamins; nacreous agents; pH stabilizers; preserving agents; and dyes.

18. Use of the composition according to any one of the claims for washing and/or conditioning keratin materials.

19. Cosmetic process for treating keratin materials, which consists in applying an effective amount of a composition according to any one of Claims 1 to 17 to the said materials, and rinsing it out after an optional leave-in time.

## Patentansprüche

1. Kosmetische Zusammensetzung vom Wasser-in-Wasser-Emulsionstyp, umfassend in einem kosmetisch unbedenklichen wäßrigen Medium:
4 bis 50 Gew.-%, bezogen auf das Gesamtgewicht der kosmetischen Zusammensetzung, mindestens eines Tensids,
mehr als 3 Gew.-%, bezogen auf das Gesamtgewicht der Zusammensetzung, mindestens eines anorganischen oder organischen wasserlöslichen Salzes, das dann, wenn es organisch ist, 1 bis 7 Kohlenstoffatome im Anion enthält,
mindestens 0,5 Gew.-%, bezogen auf das Gesamtgewicht der Zusammensetzung, mindestens eines kationischen Polymers mit einer gewichtsmittleren Molmasse von mehr als 10⁵,
in einem Gewichtsverhältnis von wasserlöslichem Salz bzw. wasserlöslichen Salzen zu kationischem Polymer bzw. kationischen Polymeren größer gleich 4,5.

2. Kosmetische Zusammensetzung nach Anspruch 1, **dadurch gekennzeichnet, daß** das Gewichtsverhältnis von wasserlöslichem Salz bzw. wasserlöslichen Salzen zu kationischem Polymer bzw. kationischen Polymeren zwischen 4,5 und 19 und vorzugsweise zwischen 4,5 und 15 liegt.

3. Kosmetische Zusammensetzung nach Anspruch 1 oder 2, **dadurch gekennzeichnet, daß** das Tensid anionisch, amphoter, nichtionisch oder kationisch ist.

4. Kosmetische Zusammensetzung nach Anspruch 3, **dadurch gekennzeichnet, daß** sie mindestens ein anionisches Tensid, gegebenenfalls in Kombination mit einem oder mehreren amphoteren oder nichtionischen Tensiden, umfaßt.

5. Kosmetische Zusammensetzung nach Anspruch 4, **dadurch gekennzeichnet, daß** sie mindestens ein anionisches Tensid und mindestens ein amphoteres Tensid umfaßt.

6. Kosmetische Zusammensetzung nach einem der Ansprüche 3 bis 5, **dadurch gekennzeichnet, daß** das anionische Tensid unter Alkalimetallsalzen, Ammoniumsalzen, Aminsalzen, Aminoalkoholsalzen oder Erdalkalimetallsalzen der folgenden Verbindungen ausgewählt ist: Alkylsulfate, Alkylethersulfate, alkylamidoethersulfate, Alkylarylpolyethersulfate, Monoglyceridsulfate; Alkylsulfonate, Alkylphosphate, Alkylamidsulfonate, Alkylarylsulfonate, α-Olefinsulfonate, Paraffinsulfonate; Alkylsulfosuccinate, Alkylethersulfosuccinate, Alkylamidsulfosuccinate; Alkylsulfoacetate; Acylsarcosinate und Acylglutamate, wobei die Alkyl- bzw. Acylgruppen aller dieser Verbindungen 6 bis 24 Kohlenstoffatome enthalten und die Arylgruppe vorzugsweise eine Phenyl- oder Benzylgruppe darstellt; C₆-C₂₄-Alkylester von Polyglykosidcarbonsäuren; Alkylsulfosuccinamate, Acylisethionate und N-Acyltaurate, wobei die Alkyl- bzw. Acylgruppen aller dieser Verbindungen 12 bis 20 Kohlenstoffatome enthalten.

7. Kosmetische Zusammensetzung nach Anspruch 6, **dadurch gekennzeichnet, daß** das anionische Tensid unter Alkylsulfaten, Alkylethersulfaten, vorzugsweise mit 2 oder 3 mol Ethylenoxid, und Alkylethercarboxylaten, wobei die Alkylgruppen 6 bis 24 Kohlenstoffatome enthalten, in Form von Natrium-, Magnesium- oder Ammoniumsalzen ausgewählt ist.

8. Kosmetische Zusammensetzung nach einem der Ansprüche 3 bis 5, **dadurch gekennzeichnet, daß** das amphotere Tensid unter (C₈-C₂₀)-Alkylbetainen, (C₈-C₂₀)-Alkylamido-(C₆-C₈)-alkylbetainen, Alkylamphomonoacetaten und Alkylamphodiacetaten ausgewählt ist.

9. Kosmetische Zusammensetzung nach einem der vorhergehenden Ansprüche, **dadurch gekennzeichnet, daß** sie das Tensid bzw. die Tenside in einer Menge von 4 bis 20 Gew.-%, bezogen auf das Gesamtgewicht der Zusammensetzung, umfaßt.

10. Kosmetische Zusammensetzung nach einem der vorhergehenden Ansprüche, **dadurch gekennzeichnet, daß** das wasserlösliche Salz unter wasserlöslichen Salzen von ein- oder zweiwertigen Metallen oder von Ammonium oder von Amin und von anorganischen Säuren oder von organischen Carbonsäuren ausgewählt ist.

11. Kosmetische Zusammensetzung nach Anspruch 10, **dadurch gekennzeichnet, daß** das wasserlösliche Salz unter Natriumchlorid, Kaliumchlorid, Calciumchlorid, Magnesiumchlorid, Ammoniumchlorid, Monoethanolaminchlorid, Natriumcitrat, Ammoniumcitrat, Magnesiumsulfat und Natriumsalzen von Phosphorsäure ausgewählt ist.

12. Kosmetische Zusammensetzung nach einem der vorhergehenden Ansprüche, **dadurch gekennzeichnet, daß** sie das wasserlösliche Salz bzw. die wasserlöslichen Salze in einer Menge von 3,1 bis 30 Gew.-%, bezogen auf das Gesamtgewicht der Zusammensetzung, umfaßt.

13. Kosmetische Zusammensetzung nach einem der vorhergehenden Ansprüche, **dadurch gekennzeichnet, daß** die gewichtsmittlere Molmasse des kationischen Polymers zwischen 10⁵ und 10⁸ liegt.

14. Zusammensetzung nach einem der vorhergehenden Ansprüche, **dadurch gekennzeichnet, daß** das kationische Polymer unter Celluloseetherderivaten mit quaternären Ammoniumgruppen, kationischen Cyclopolymeren, mit einem 2,3-Epoxypropyltrimethylammoniumsalz modifizierten Guargummen und quaternären Polymeren von Vinylpyrrolidon und Vinylimidazol ausgewählt ist.

15. Zusammensetzung nach einem der vorhergehenden Ansprüche, **dadurch gekennzeichnet, daß** sie das kationische Polymer bzw. die kationischen Polymere in einer Menge von 0,5 bis 10 Gew.-%, bezogen auf das Gesamtgewicht der Zusammensetzung, umfaßt.

16. Zusammensetzung nach einem der vorhergehenden Ansprüche, **dadurch gekennzeichnet, daß** das kosmetisch unbedenkliche wäßrige Medium aus Wasser oder einer Mischung von Wasser und mindestens einem organischen Lösungsmittel besteht.

17. Zusammensetzung nach einem der vorhergehenden Ansprüche, **dadurch gekennzeichnet, daß** sie außerdem mindestens ein unter organisch modifizierten oder nicht organisch modifizierten, linearen, verzweigten oder cyclischen, flüchtigen oder nichtflüchtigen Silikonen; natürlichen oder synthetischen Verdickungsmitteln oder Viskositätsreglern; C₁₂-C₃₀-Fettalkoholen; Wachsen; öligen Fettestern; mineralischen oder synthetischen Ölen; Vitaminen und Provitaminen; Glanzmitteln; pH-Stabilisatoren, Konservierungsmitteln und Farbmitteln ausgewähltes Additiv umfaßt.

18. Verwendung der Zusammensetzung nach einem der Ansprüche zum Waschen und/oder Konditionieren von Keratinmaterialien.

19. Verfahren zur kosmetischen Behandlung von Keratinmaterialien, bei dem man eine wirksame Menge einer Zusammensetzung nach einem der Ansprüche 1 bis 17 auf die Materialien aufbringt und nach einer fakultativen Einwirkungszeit ausspült.
